# EUROPÄISCHE PATENTSCHRIFT

(11) **EP 0 656 212 B1**
(45) Veröffentlichungstag und Bekanntmachung des Hinweises auf die Patenterteilung: **24.01.2001**
(21) Anmeldenummer: 94117612.5
(22) Anmeldetag: 08.11.1994
(51) Int. Cl.: A61K 38/13, A61K 47/14, A61K 47/10

(54) **Cyclosporin enthaltende flüssige Zubereitungen und Verfahren zu ihrer Herstellung**
Liquid compositions containing cyclosporin and process for their preparation
Compositions liquides comprenant de la cyclosporine et leur procédé de préparation

(30) Priorität: 30.11.1993 DE 4340781
(43) Veröffentlichungstag der Anmeldung: 07.06.1995
(73) Patentinhaber: Novartis AG, 4058 Basel (CH); Novartis-Erfindungen Verwaltungsgesellschaft m.b.H., 1235 Wien (AT)
(72) Erfinder: Walch, Hatto, Dr., D-88471 Laupheim-Baustetten (DE); Fleck, Monika, Dr., D-88471 Laupheim (DE); Neuer, Klaus, D-88477 Schwendi-Grossschaffhausen (DE)

(56) Entgegenhaltungen:
- WO-A-93/18746

## Beschreibung

Die Erfindung betrifft Cyclosporin, insbesondere Cyclosporin A enthaltende flüssige Zubereitungen zur oralen oder parenteralen Verabreichung und ein Verfahren zu ihrer Herstellung, wobei Aerosol-Druckgaspackungen ausgenommen sind.

Cyclosporine sind cyclische Oligopeptide mikrobiologischer Herkunft, die insbesondere als Immunsuppresiva verwendet werden.

Cyclosporine, insbesondere Cyclosporin A, werden in der Organtransplantation zur Verhinderung der Abstoßung des transplantierten Organs eingesetzt.

Darüber hinaus ist bekannt, daß Cyclosporine eine entzündungshemmende und antiparasitäre Wirkung aufweisen.

Der Einsatz der Cyclosporine ist daher nicht nur auf Immunsuppresiva beschränkt, sondern betrifft verschiedene Autoimmunerkrankungen und entzündliche Zustände, insbesondere entzündliche Zustände, in denen Autoimmunprozesse eine Rolle spielen. Hierzu zählen arthritische Erkrankungen, wie z.B. rheumatoide Arthritis und rheumafische Erkrankungen.

Als antiparasitäre Mittel können Cyclosporine zur Behandlung von Protozoeninfektionen, wie z.B. Malaria, eingesetzt werden.

Mit den bislang in der Praxis eingesetzten Cyclosporinformulierungen mußten jedoch schwerwiegende Nebenwirkungen, insbesondere nierenschädigende, in Kauf genommen werden.

Cyclosporine sind Substanzen mit stark hydrophobem Charakter. Aufgrund der schlechten Wasserlöslichkeit ist es schwierig, Cyclosporine mit den üblichen pharmazeutischen Hilfsstoffen zu Zubereitungen mit ausreichender Bioverfügbarkeit zu verarbeiten.

Bislang vorgeschlagene cyclosporinhaltige pharmazeutische Zubereitungen basieren auf der Verwendung eines Alkohols und/oder Ölen oder ähnlichen Vehikeln in Verbindung mit einem oberflächenaktiven Stoff.

Die US-A-4 388 307 schlägt die Lösung des Cyclosporins in einem Gemisch aus mit Polyethylenglykol gebildeten Umesterungsprodukten unterschiedlicher Öle (z.B. Labrafil® M 1499 CS) sowie von Ethanol und einem Pflanzenöl vor. Die so erhaltenen Produkte sind in Folge ihres Ölgehaltes für eine intravenöse Verabreichung jedoch ungeeignet. Sie können nur auf subkutanem oder intramuskulärem Weg verabreicht werden.

Gemäß der Sandimmun® Produktinformation (Kapitel XII, Sandoz-Pharma, Basel, 1984) wird Cyclosporin in einem Gemisch von polyoxyethyliertem Rizinusöl (Cremophor® EL, BASF) und Ethanol gelöst. Der Nachteil dieser Zubereitungen besteht darin, daß sie von den Patienten schlecht vertragen werden, da oft anaphylaktische Reaktionen auftreten (KAHAN et al., Lancet, 1984, I:52; LEUNISSEN, K.M. et al., Lancet, 1985, I:636).

Die WO 92/09299 betrifft orale flüssige pharmazeutische Zubereitungen, die ein Cyclosporin in einer Mischung aus einem hydrophilen Lvsungsmittel und einem oberflächenaktiven Stoff enthalten. Als oberflächenaktive Substanzen werden Polyoxyethylen-Polyoxypropylen-Blockpolymere(Poloxamere) mit Mol.-Gew. von 1.000 bis 15.500 eingesetzt. Nachteilig bei dieser Formulierung ist die Präzipitation des Wirkstoffs bei Kontakt mit wässrigen Lösungen. Aufgrund der Solubilisationsfähigkeit der Polyoxamere sind diese Formulierungen für eine parenterale Verabreichung ungeeignet.

Die WO 93/18746 offenbart flüssige, unter anderem Cyclosporin enthaltende Zubereitungen in Aerosol-Druckgaspackungen.

Aufgabe der Erfindung ist es, homogene, flüssige Zubereitungen mit dem in Wasser schwer bzw. unlöslichen Cyclosporin bzw. Cyclosporinen zur Verfügung zu stellen, die sich in jedem Mengenverhältnis mit Wasser verdünnen lassen und dabei klare, stabile Lösungen bilden.

Eine weitere Aufgabe der Erfindung ist es, Formulierungen zur Verfügung zu stellen, die zu einer besseren Bioverfügbarkeit des Wirkstoffs führen und damit eine Reduktion der Menge an zu verabreichendem Wirkstoff erlauben.

Die Anmelderin hat nun überraschend gefunden, daß die vorstehenden Aufgaben durch eine Lösung, die Cyclosporin gelöst in einem Gemisch aus einem nichtionogenen Emulgator vom Typ Polyoxyethylen-Glycerin-Fettsäuremonoester und ein- und/oder mehrwertigen Alkoholen gelöst werden können, wobei die Lösungen stabil sind, gut verträglich sind und eine verbesserte Bioverfügbarkeit besitzen und sowohl oral als auch parenteral verabreicht werden können.

Genauer gesagt, betrifft die Erfindung flüssige pharmazeutische Zubereitungen zur oralen oder parenteralen Verabreichung, die Cyclosporin als Wirkstoff in Kombination mit einem Polyoxyethylen-Glycerin-Fettsäuremonoester und ein- und/oder mehrwertigen Alkohol(en) enthalten, wobei Aerosol-Druckgaspackungen ausgeschlossen sind.

Bei den Polyoxyethylen-Glycerin-Fettsäuremonoester (PGFME) handelt es sich um nichtionische Emulgatoren, insbesondere um diejenigen, die unter der Handelsbezeichnung Tagat® erhältlich sind. Bevorzugte Verbindungen daraus sind die Monoester der Laurin-, Stearin-, Olein- und Isostearinsäure. Besonders bevorzugt sind die Monoester der Oleinsäure und der Laurinsäure, die unter der Bezeichnung Tagat® O und Tagat® L kommerziell erhältlich sind. Der HLB-Wert des verwendeten Emulgators liegt im Bereich 10 bis 20, bevorzugt 14 bis 17.

Die erfindungsgemäßen Lösungskonzentrate enthalten, bezogen auf ein Gew.-Teil Wirkstoff 1 bis 20 Gew.-Teile des PGFME und 0,5 bis 20 Gew.-Teile des ein- und/oder mehrwertigen Alkohols(e), bevorzugt 10 bis 20 Teile PGFME und 2 bis 10 Teile Alkohol und insbesondere 12 bis 18 Teile PGFME und 3 bis 6 Teile Alkohol.

Zur Verwendung in den erfindungsgemäßen Zubereitungen sind alle bekannten natürlichen und synthetischen Cyclosporine einschließlich ihrer Analoga und Derivate geeignet. Beispiele für derartige Cyclosporine finden sich z.B. in der DE-OS 40 03 844 und DE-OS 40 05 190. Bevorzugt wird Cyclosporin A verwendet.

Die Wirkstoffkonzentrationen der erfindungsgemäßen Lösungskonzentrate liegen bei 20 bis 200 mg/ml, vorzugsweise bei 50 bis 100 mg/ml.

Die Alkoholkomponenten sind ein- und/oder mehrwertige Alkohole als Einzelsubstanzen oder in beliebigen Mischungen, wie z.B. Ethanol, Propylenglykol und/oder Polyethylenglykole mit einem Mol.-Gewicht bis 600. Vorteilhafterweise wird Ethanol und/oder Propylenglykol verwendet.

Fakultativ können die erfindungsgemäßen Zubereitungen darüber hinaus weitere intravenös anwendbare Träger- und/oder Hilfsstoffe bzw. die zur oralen Verabreichung bestimmten Zubereitungen übliche pharmazeutische Zusatzstoffe, wie z.B. Geschmackskorrigenzien, Verdünnungsmittel, Konservierungsmittel, Isotonierungsmittel etc enthalten.

Die anschließenden Beispiele dienen der näheren Erläuterung der Erfindung, ohne jedoch darauf beschränkt zu sein.

### Beispiel 1:

70 g Polyoxyethylen-Glycerin-Monooleat (Tagat® O) werden mit 30 g Propylenglykol gemischt. In dem so erhaltenen Gemisch werden bei Raumtemperatur 5 g Cyclosporin A gelöst. Die Lösung wird mit Propylenglykol auf 100 ml aufgefüllt und durch Rühren homogenisiert. Je nach Verwendungszweck wird das so hergestellte Lösungskonzentrat in Flaschen oder Ampullen abgefüllt bzw. zu Weichgelatinekapseln weiterverarbeitet. Bei Herstellung von Parenteralia müssen Herstellung und Abfüllung unter Sterilbedingungen erfolgen. Das so erhaltene Konzentrat wird vor der therapeutischen Anwendung durch Verdünnung mit Wasser oder wässrigen Lösungen auf den gewünschten Wirkstoffgehalt eingestellt.

### Beispiel 2:

80 g Polyoxyethylen-Glycerin-Monolaurat (Tagat® L2) werden mit 10 g 96 Vol.-%igem Ethanol gemischt. In dieser Mischung werden bei Raumtemperatur 5 g Cyclosporin A unter Rühren gelöst. Die so erhaltene Lösung wird mit 96 Vol.-%igem Ethanol auf 100 ml aufgefüllt und durch Rühren homogenisiert. Die weitee Vorgehensweise erfolgt wie in Beispiel 1.

### Beispiel 3:

30 g Polyoxyethylen-Glycerin-Monolaurat (Tagat® L2) werden mit 65 g Propylenglykol gemischt. In diesem Gemisch werden 5 g Cyclosporin A gelöst. Die so erhaltene Zubereitung wird, wie bei Beispiel 1 beschrieben, weiterverarbeitet.

### Beispiel 4:

70 g Polyoxyethylen-Glycerin-Monostearat (Tagat® S) werden mit 30 g 96 Vol.-% igem Ethanol vermischt und darin unter Rühren 5 g Cyclosporin A gelöst. Das so erhaltene Konzentrat wird wie im Beispiel 1 weiterbearbeitet.

### Beispiel 5:

75 g Polyoxyethylen-Glycerin-Monostearat (Tagat® S) werden mit 10 g 96 Vol.-%igem Ethanol und 10 g Propylenglykol gemischt und in dieser Mischung unter Rühren 5 g Cyclosporin A gelöst. Die Lösung wird mit 96 Vol.-%igem Ethanol auf 100 ml aufgefüllt und durch Rühren homogenisiert. Die weitere Bearbeitung ist in Beispiel 1 beschreiben.

### Beispiel 6:

60 g Polyoxyethylen-Glycerin-Monooleat (Tagat® O) werden mit 20 g 96 Vol.-%igem Ethanol gemischt und darin unter Rühren 5 g Cyclosporin A gelöst. Die Lösung wird mit 96 Vol.-%igem Ethanol auf 100 ml aufgefüllt. Die weitere Bearbeitung ist in Beispiel 1 beschrieben.

### Beispiel 7:

88 g Polyoxyethylen-Glycerin-Monooleat (Tagat® O) werden mit 10 g Propylenglykol gemischt und darin unter Rühren 10 g Cyclosporin A gelöst. Die Lösung wird mit Propylenglykol auf 100 ml aufgefüllt und weiter, wie unter Beispiel 1 beschrieben, bearbeitet.

Die erfindungsgemäß hergestellten Lösungskonzentrate werden in Flaschen oder Ampullen abgefüllt und vor der therapeutischen Anwendung auf den gewünschten Wirkstoffgehalt verdünnt. Je nach gewünschtem Wirkstoffgehalt werden die Konzentrate in einem Gewichtsverhältnis von 1:10 bis 1:100 verdünnt. Als Verdünnungsmittel können Wasser oder wässrige Lösungen, wie z.B. physiologische Kochsalzlösungen, Glucose-, Dextran-, Fructose- oder Mannit-Lösungen, verwendet werden.

Lösungskonzentrate zur oralen Verabreichung können auch in Weichgelatinekapseln abgefüllt werden.

Die erfindungsgemäßen Zubereitungen zeigten nach 6-monatiger Lagerung im Streßtest bei Temperaturen von -18°C bis 60°C keine Ausfällungen, Zersetzungen oder sonstige Veränderungen.

### Bioverfügbarkeit:

Für die BV-Untersuchungen der erfindungsgemäßen Zusammensetzungen wurde eine Gruppe von Beagle-Hunden eingesetzt. Die Applikation der Prüfpräparate erfolgte oral mittels Schlundsonde am nüchternen Tier. Den Tieren wurde zu definierten Zeitpunkten Blut aus der Vena saphena entnommen und in entsprechenden Kunststoffröhrchen mit EDTA-Zusatz gesammelt. Die Blutproben wurden bis zur Bestimmung bei -18°C gelagert. Die Bestimmung des Cyclosporins erfolgte im Vollblut mittels Fluoreszenz-Polarisations-Immunoassay (FPIA).

Die Flächen unter den Kurven (AUC), bei denen die Blutarzneistoffkonzentration gegen die Zeit aufgetragen wird, wurden nach der Trapezregel berechnet. Die durchschnittlichen AUC-Werte der erfindungsgemäßen Zusammensetzung liegen im Vergleich zu der im Handel befindlichen Sandimmun®-Trinklösung, die in gleicher Weise, bei gleicher Dosierung mit den gleichen Hunden, ermittelt wurde, wesentlich höher.

Die Bioverfügbarkeiten der erfindungsgemäßen pharmazeutischen Zubereitungen sind je nach Zusammensetzung überraschenderweise ca. 40 bis 70% höher als bei der bislang am Markt befindlichen Formulierung.

Durch dieses überraschende Ergebnis ist es möglich, die Dosis des Wirkstoffes zu reduzieren und somit die schwerwiegenden Nebenwirkungen der bisherigen Formulierungen, insbesondere Nebenwirkungen nephrotoxischer Art, drastisch zu senken.

## Patentansprüche

1. Flüssige Zubereitungen zur oralen oder parenteralen Verabreichung, enthaltend ein Cyclosporin als Wirkstoff in Kombination mit einem Polyoxyethylen-Glycerin-Fettsäuremonoester und einem oder mehreren ein- und/oder mehrwertigen Alkohol(en), wobei Aerosol-Druckgaspackungen ausgenommen sind.

2. Flüssige Zubereitungen nach Anspruch 1, dadurch **gekennzeichnet,** daß sie als Polyoxyethylen-Glycerin-Fettsäuremonoester Ester der Laurin-, Stearin-, Olein- oder Isostearinsäure enthalten.

3. Flüssige Zubereitungen nach Anspruch 2, dadurch **gekennzeichnet,** daß der Polyoxyethylen-Glycerin-Fettsäuremonoester eine Monoester der Olein- oder Laurinsäure ist.

4. Pharmazeutische Zubereitungen nach einem der Ansprüche 1 bis 3, dadurch **gekennzeichnet,** daß die Komponenten Wirkstoff, PGFME und Alkohol im Verhältnis 1:1 - 20:0,5 - 20, bevorzugt 1:10 - 20:2 - 10 und insbesondere 1:12 -18:3 - 6 vorliegen.

5. Pharmazeutische Zubereitungen nach einem der Ansprüche 1 bis 4, dadurch **gekennzeichnet,** daß es sich bei der Alkoholkomponente um Ethanol, Propylenglykol und/oder Polyethylenglykol mit einem Mol.-Gewicht bis etwa 600 handelt oder um Mischungen davon.

6. Pharmazeutische Zubereitungen nach einem der Ansprüche 1 bis 5, dadurch **gekennzeichnet,** daß die Wirkstoffkonzentrationen 20 bis 200 mg/ml, bevorzugt 50 bis 100 mg/ml betragen.

7. Verfahren zur Herstellung der flüssigen Zubereitungen nach einem der Ansprüche 1 bis 6, dadurch **gekennzeichnet,** daß man die Komponenten bei Temperaturen von 20 bis 50°C mischt und anschließend in geeignete Behältnisse abfüllt.

8. Pharmazeutische Zubereitung nach einem der Ansprüche 1 bis 6 zu Weichgelatinekapseln verarbeitet.

9. Verwendung einer pharmazeutischen Zubereitung nach einem der Ansprüche 1 bis 6 oder Anspruch 8 für die Herstellung eines Arzneimittels für die therapeutische Anwendung Organtransplantation, Entzündungshemmung, parasitäre Erkrankungen, arthritische Erkrankungen, rheumatische Erkrankungen.

10. Verwendung einer pharmazeutischen Zubereitung nach einem der Ansprüche 1 bis 6 für die Herstellung eines Arzneimittels für die therapeutische Anwendung Organtransplantation wobei die pharmazeutische Zubereitung zu Weichgelatinekapseln verarbeitet ist.

## Claims

1. Liquid preparations for oral or parenteral administration, comprising cyclosporin as the active ingredient in combination with a polyoxyethylene glycerol fatty acid monoester and one or more monovalent and/or polyvalent alcohol(s), whereby compressed gas aerosol dispensers are excluded.

2. Liquid preparations according to claim 1, which contain esters of lauric, stearic, oleic or isostearic acid as the polyoxyethylene glycerol fatty acid monoester.

3. Liquid preparations according to claim 2, in which the polyoxyethylene glycerol fatty acid monoester is a monoester of oleic or lauric acid.

4. Pharmaceutical preparations according to one of claims 1 to 3, in which the components: active ingredient, PGFME and alcohol are present in a ratio of 1:1-20:0.5-20, preferably 1:10-20:2-10, especially 1:12-18:3-6.

5. Pharmaceutical preparations according to one of claims 1 to 4, in which the alcohol component in question is ethanol, propylene glycol and/or polyethylene glycol with a molecular weight of up to ca. 600, or mixtures thereof.

6. Pharmaceutical preparations according to one of claims 1 to 5, in which the active ingredient concentration is 20 to 200 mg/ml, preferably 50 to 100 mg/ml.

7. A method of preparing the liquid preparations according to one of claims 1 to 6, in which the components are mixed at temperatures of 20 to 50°C and then filled into suitable containers.

8. Pharmaceutical preparation according to one of claims 1 to 6, processed into soft gelatin capsules.

9. Usage of a pharmaceutical preparation according to one of claims 1 to 6 or 8 in the preparation of a medicament for therapeutic usage, such as for organ transplantations, as anti-inflammatory agents, for parasitic diseases, arthritic diseases, rheumatic diseases.

10. Usage of a pharmaceutical preparation according to one of claims 1 to 6 for the preparation of a medicament for therapeutic usage, such as for organ transplantations, whereby the pharmaceutical preparation is processed into soft gelatin capsules.

## Revendications

1. Des compositions liquides pour l'administration par voie orale ou parentérale, contenant une cyclosporine comme substance active en association avec un monoester d'un acide gras du polyoxyéthylène et du glycérol et un ou plusieurs mono- et/ou polyalcool(s), les aérosols avec un conditionnement sous gaz comprimé étant exclus.

2. Des compositions liquides selon la revendication 1, caractérisées en ce qu'elles contiennent comme monoester d'un acide gras du polyoxyéthylène et du glycérol, un ester de l'acide laurique, de l'acide stéarique, de l'acide oléique ou de l'acide isostéarique.

3. Des compositions liquides selon la revendication 2, caractérisées en ce que le monoester d'un acide gras du polyoxyéthylène et du glycérol est un monoester de l'acide oléique ou de l'acide laurique.

4. Des compositions pharmaceutiques selon l'une quelconque des revendications 1 à 3, caractérisées en ce que le rapport des composants substance active, PGFME et alcool sont de 1:1 - 20:0,5-20, de préférence de 1:10 - 20:2 -10 et en particulier de 1:12 - 18:3-6.

5. Des compositions pharmaceutiques selon l'une quelconque des revendications 1 à 4, caractérisées en ce que le composant alcool est de l'éthanol, du propylèneglycol et/ou du polyéthylèneglycol ayant un poids mol. jusqu'à environ 600 ou leurs mélanges.

6. Des compositions pharmaceutiques selon l'une quelconque des revendications 1 à 5, caractérisées en ce que les concentrations en matière active vont de 20 à 200 mg/ml, de préférence de 50 à 100 mg/ml.

7. Un procédé de préparation de compositions liquides selon l'une quelconque des revendications 1 à 6, caractérisé en ce qu'on mélange les composants à des températures de 20 à 50°C et on les introduit ensuite dans des récipients appropriés.

8. Une composition pharmaceutique selon l'une quelconque des revendications 1 à 6, pour la mise sous forme de capsules de gélatine molles.

9. L'utilisation d'une composition pharmaceutique selon l'une quelconque des revendications 1 à 6 ou selon la revendication 8, pour la préparation d'un médicament pour l'utilisation thérapeutique dans les greffes d'organes, l'inhibition des inflammations, les maladies parasitaires, les maladies arthritiques, les maladies rhumatismales.

10. L'utilisation d'une composition pharmaceutique selon l'une quelconque des revendications 1 à 6, pour la préparation d'un médicament pour l'utilisation thérapeutique dans les greffes d'organes, la composition pharmaceutique étant mise sous forme de capsules de gélatine molles.
